# EUROPEAN PATENT APPLICATION

(11) **EP 4 179 959 A1**
(43) Date of publication of application: **17.05.2023**
(21) Application number: 22207226.6
(22) Date of filing: 14.11.2022
(51) Int. Cl.: A61B 3/15, A61B 3/10

(54) **GYRO SENSOR TILTING MODULE OF VISION SCREENER**

(30) Priority: 16.11.2021 KR 20210157895
(71) Applicant: Huvitz Co., Ltd., Anyang-si, Gyeonggi-do 14055 (KR)
(72) Inventor: LEE, Young Woo, 14055 Gyeonggi-do (KR); MO, Jae Hong, 14055 Gyeonggi-do (KR)
(74) Representative: IPAZ

(57) **Abstract**

Disclosed is to a gyro sensor tilting module of a vision screener for examining eye of subjects of various heights. The gyro sensor tilting module of a vision screener comprises: a gyro sensor 10 for detecting a tilting angle of the vision screener; a driving motor 20 for tilting the optical unit 40 by the tilting angle in accordance with the tilting angle detected by the gyro sensor 10; and the optical unit 40 which is tilted by the driving motor 20 to be aligned horizontally with an eye of a subject to be examined for measuring the eye.

## Description

### CROSS-REFERENCE TO RELATED APPLICATION

This application claims benefit of priority to Korean Patent Application No. 10-2021-0157895 filed on November 16, 2021, the entire contents of which are incorporated herein by reference.

### FIELD OF THE DISCLOSURE

The present invention relates to a gyro sensor tilting module of a vision screener, and more particularly, to a gyro sensor tilting module of a vision screener for examining eye of subjects (patients) of various heights.

### BACKGROUND

A vision screener is a simple eye testing device for early diagnosing potential vision problems and/or eye disorders. The vision screener is conventionally used for initially identifying individuals having vision problems rather than for complete eye testing performed by an ophthalmologist and/or optometrist. Namely, the vision screener is generally used for a preliminary examination of an eye rather than a complete or comprehensive eye examination. The vision screener is a hand-held device, and can be used outside of a hospital or an eye examining room. The eye test using the vision screener is fast, simple, inexpensive. Therefore, the vision screener can be used to identify and examine subjects of various age groups, such as infants, children, and teenagers, who would be currently experiencing myopia. The vision screener is often used for regular checkups for children. For example, a vision screening is disclosed in international patent publication No. WO 2021/164864.

However, in the case of an eye testing with a conventional vision screener, the vision screener should be located horizontally at the eye level of a subject to be examined. Thus, when the height of the subject (for example, children) is short, the examiner, for example, an optometrist cannot properly monitor the LCD screen of the vision screener which located at a lower position than his/her eye level, and the optometrist should bend his/her waist or knees to monitor the LCD screen of the vision screener. Thus, it is uncomfortable for the optometrist.

### SUMMARY OF THE INVENTION

### TECHNICAL OBJECTS

It is an object of the present invention to provide a gyro sensor tilting module of vision screener, which can examine an eye of a subject at eye level of the subject without bending the optometrist's waist or knees.

It is another object of the present invention to provide a gyro sensor tilting module of vision screener capable of maintaining an optical unit horizontal even when the vision screener is tilted.

### TECHNICAL SOLUTION

In order to achieve the objects above, the present invention provides a gyro sensor tilting module of a vision screener, comprising: a gyro sensor 10 for detecting a tilting angle of the vision screener; a driving motor 20 for tilting the optical unit 40 by the tilting angle in accordance with the tilting angle detected by the gyro sensor 10; and the optical unit 40 which is tilted by the driving motor 20 to be aligned horizontally with an eye of a subject to be examined for measuring the eye.

### EFFECTS OF THE INVENTION

The gyro sensor tilting module of vision screener in accordance with the present invention can maintain the optical unit horizontal when the vision screener is tilted, so that the optometrist can examine an eye of a subject at eye level of the subject without bending the examiner's the waist or the knees to examine the subject.

### BRIEF DESCRIPTION OF THE DRAWINGS

Fig. 1a is a side view for illustrating the gyro sensor tilting module located within a vision screener in accordance with one embodiment of the present invention.

Fig. 1b is a perspective view for illustrating the gyro sensor tilting module located within a vision screener in accordance with one embodiment of the present invention.

Fig. 2 is a view for illustrating the structure of the gyro sensor tilting module of vision screener in accordance with one embodiment of the present invention.

Fig. 3 is a view for illustrating an operation of the gyro sensor tilting module of the vision screener in accordance with one embodiment of the present invention.

Fig. 4 is a view for illustrating the tilting angle (inclination) of the gyro sensor tilting module of vision screener in accordance with one embodiment of the present invention.

Fig. 5 is a view for illustrating a comparison of the eye levels (directions) of an examiner when the vision screener in accordance with an embodiment of the present invention and the conventional vision screener are used.

### DETAILED DESCRIPTION OF THE PREFERRED EMBODIMENTS

Hereinafter, the present invention will be described in detail with reference to the accompanying drawings.

The vision screener can be a refractometer for optometry. The vision screener in accordance with the present invention examines an eye of a subject with features similar to those of a conventional vision screener. For example, the vision screener of the present invention may include an optical unit (measurement device) for measuring an eye of a subject (for example, a patient), an analysis unit for analyzing data measured by the optical unit, and a display unit for displaying results analyzed by the analysis unit. The gyro sensor tilting module of the present invention is used for the vision screener of the present invention

In the case of a conventional vision screener, if the position of the vision screener is lowered to measure the eyes of a short subject, the examiner, for example, the optometrist cannot directly see the LCD screen of the display unit of the vision screener and thus cannot properly perform the eye examination. In the gyro sensor tilting module, namely, the vision screener in accordance with the present invention, when the vision screener is tilted according to the position of the subject's eye to be examined, the optical unit is tilted to be aligned with the subject's eye to be examined by the tilting module of the vision screener. Therefore, when the vision screener is tilted, the optical unit is positioned horizontally, and it is possible to properly measure the subject's eye.

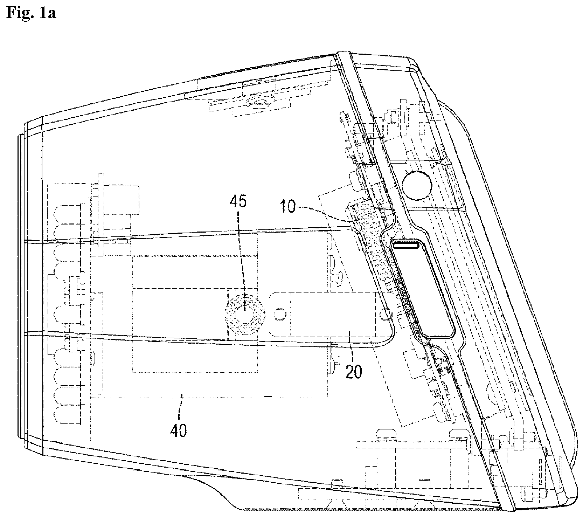

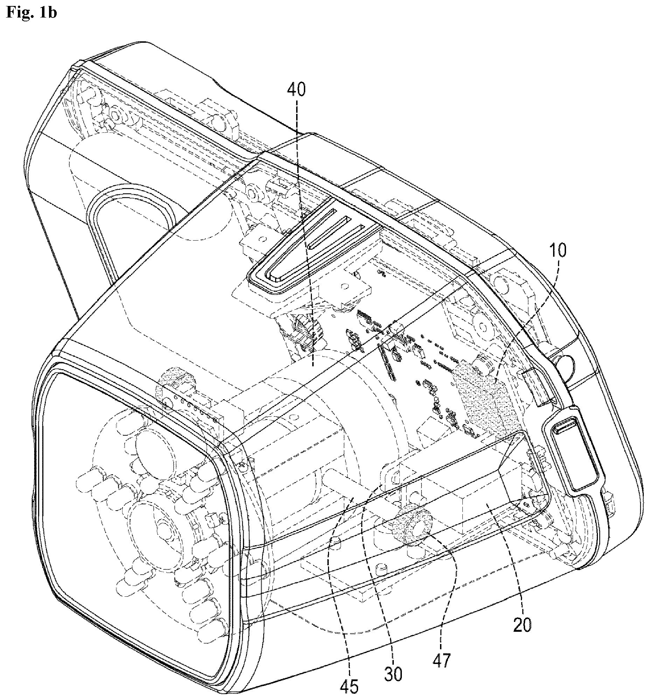

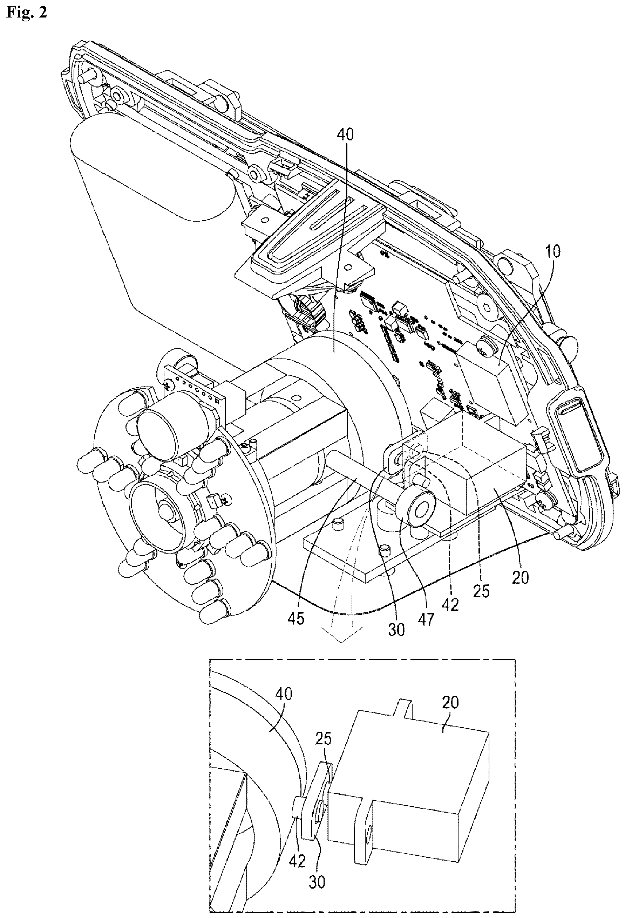

Fig. 1a is a side view for illustrating the gyro sensor tilting module located within a vision screener in accordance with one embodiment of the present invention. Fig. 1b is a perspective view for illustrating the gyro sensor tilting module located within a vision screener in accordance with one embodiment of the present invention. Fig. 2 is a view for illustrating the structure of the gyro sensor tilting module of vision screener in accordance with one embodiment of the present invention. As shown in Figs. 1a, 1b and 2, the vision screener, specifically, the gyro sensor tilting module of the vision screener in accordance with the present invention comprises a gyro sensor 10 for detecting a tilting angle of the vision screener; a driving motor 20 for tilting the optical unit 40 by the tilting angle in accordance with the tilting angle detected by the gyro sensor 10, and the optical unit 40 which is tilted by the driving motor 20 to be aligned horizontally with an eye of a subject to be examined for measuring the eye.

The driving motor 20 may include a motor shaft 25 which is rotated in accordance with the tilting angle detected by the gyro sensor 10, and a rotation guide 30 mounted to the motor shaft 25 and rotated in accordance with the tilting angle with the motor shaft 25. The driving motor 20 may be located below the gyro sensor 10. One end of the rotation guide 30 is connected to an optical unit connecting pin 42 which is mounted on the optical unit 40. Specifically, the optical unit connecting pin 42 is inserted into a hole formed on the end of the rotation guide 30 and rotates in the hole. The optical unit 40 is rotatably mounted in the vision screener via an optical unit rotation shaft 45. Specifically, the optical unit rotation shaft 45 may be installed in a frame of the vision screener and the optical unit 40 may be rotatably mounted on the optical unit rotation shaft 45.

The gyro sensor 10 detects the position (movement) of a target object, specifically, detects a tilting angle of the vision screener. Specifically, the examiner tilts the vision screener according to the height of the eye to be examined. When the vision screener is tilted, the tilting angle (movement) of the vision screener is detected with the gyro sensor 10, and a signal corresponding to the detected tilting angle (movement) is transmitted to the driving motor 20, and thereby the optical unit 40 is tilted to be aligned horizontally with the eye to be examined.

The gyro sensor 10 detects the movement (tilt) of the vision screener and transmits the sensed signal to the driving motor 20 so that the position of the optical unit 40 is maintained to be horizontal with the eye to be examined.

In order to control the position of the optical unit 40, the detected signal is transmitted to the driving motor 20. The transmission of the detected signal and the driving of the driving motor 20 can be controlled by a control unit (not shown in figures). For example, when there is a change in the signal value produced from the gyro sensor 10 for a certain period of time, it can be determined that there is a movement (tilting). Alternatively, when the output signal value is not changing (namely, constant), it can be determined that there is no movement. Namely, when the vision screener is tilted according to the position of the eye, the driving motor 20 is driven by the tilting signal.

A signal according to the tilting movement of the vision screener produced by the gyro sensor 10 is transmitted to the driving motor 20. According to the transmitted signal, the driving motor 20 is driven so that the optical unit 40 can be maintained to be horizontal with the eye to be examined. Specifically, the driving motor 20 mechanically rotates the motor shaft 25, the rotation guide 30, and the optical unit connection pin 42.

As shown in Fig. 2, the motor shaft 25 is connected to the driving motor 20. Specifically, the motor shaft 25 is located on one end of the driving motor 20, more specifically, on the rear surface of the driving motor 20 facing with the optical unit 40. When the driving motor 20 is driven according to the signal transmitted from the gyro sensor 10, the motor shaft 25 is rotated by the driving power.

The rotation guide 30 may be fixedly mounted to the motor shaft 25, specifically, to the other end of the motor shaft 25 which is opposite to the end connected to the driving motor 20. When the motor shaft 25 is rotated, the rotation guide 30 and the optical unit connecting pin 42 connected to the rotation guide 30 are rotated together, and the optical unit 40 is tilted.

One end of the optical unit connecting pin 42 is rotatably connected to one end of the rotation guide 30 and the other end of the optical unit connecting pin 42 is fixedly connected to one end of the optical part 40 (i.e., on the opposite side of the lens for observing the eye, the rear side).

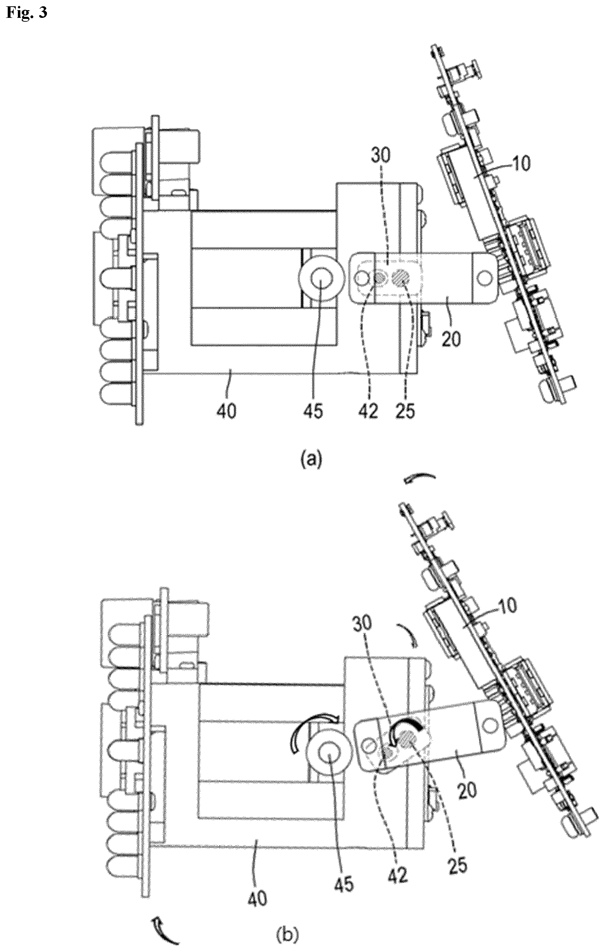

Fig. 3 is a view for illustrating the driving of the gyro sensor tilting module of the vision screener in accordance with one embodiment of the present invention. Specifically, Fig. 3(a) is a view for showing the direction of a force generated while the driving motor 20 is driven by a signal sensed by the gyro sensor 10. Fig. 3(b) is a view for showing the driving of the tilting module of the vision screener according to the direction of the generated force. As shown in Fig. 3, when the gyro sensor 10 detects the tilting movement of the vision screener according to the eye height, the detected signal value is transmitted to the driving motor 20, and the driving motor 20 is driven thereby. When the driving motor 20 is driven, the motor shaft 25 of the driving motor 20 is rotated. Then, the rotation guide 30 fixed to the motor shaft 25 also is rotated. When the rotation guide 30 is rotated, (i) the optical unit connecting pin 42 located at one end of the rotation guide 30 and (ii) the optical unit 40 are also moved, specifically rotated by the tilting angle with a rotation axis of the optical unit rotation shaft 45.

For example, as shown in Fig. 3, when the motor shaft 25 is moved (rotated) in a counterclockwise direction, the rotation guide 30 fixed to the motor shaft 25 also rotates in a counterclockwise direction, and the optical unit connecting pin 42 is rotated in a clockwise direction. At this time, the rotation axis of the optical unit connecting pin 42 is the optical unit rotation shaft 45. On the contrary, when the rotation guide 30 is rotated in a clockwise direction, the optical unit connecting pin 42 is rotated in a counterclockwise direction. Thereby, the position of the optical unit 40 can be maintained horizontal.

With this structure, the optical unit connecting pin 42 and the optical unit 40 are driven (rotated) at a predetermined angle with the rotation axis of the optical unit rotation shaft 45. Thereby, the position of the optical unit 40 can be maintained horizontal. In other words, the optical unit 40 is inversely tilted with the rotation axis of the optical unit rotation shaft 45 according to the tilting angle of the vision screener (namely, the gyro sensor 10). That is, according to the tilted position of the vision screener, the optical unit 40 also is rotated at a predetermined angle, and the position of the optical unit 40 is maintained to be horizontal.

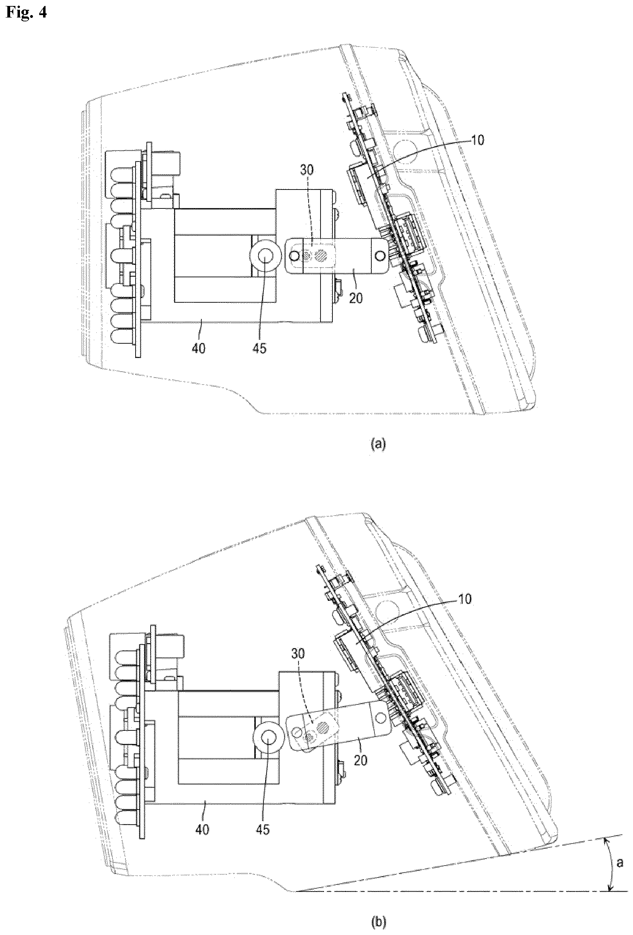

Fig. 4 is a view for illustrating the driving angle (inclination) of the gyro sensor tilting module of the vision screener in accordance with one embodiment of the present invention. Fig. 4(a) is a view for showing a tilting angle of 0° of the vision screener. Fig. 4(b) is a view for showing that the tilting angle (a) of the vision screener. As shown in Fig. 4 (b), when the vision screener is tilted by the tilting angle (a), the gyro sensor 10 detects the movement of the vision screener, and the optical unit 40 is also be driven (rotated) by the tilting angle (a) so that the position of the optical unit 40 can be maintained in horizontal with respect to the eye to be examined. Due to the limitation of the internal space of the vision screener, the rotatable inclination angle of the optical unit 40 and the vision screener may be between 0 to 10°, and may have a maximum tilting angle of 10°. Specifically, the optical unit 40 is rotated by the angle of between 0 to 10°.

The gyro sensor tilting module of vision screener in accordance with the present invention may further comprise a bearing 47 at each end of the optical unit rotation shaft 45 in order to rotate the optical unit rotation shaft 45 and the optical unit 40 with the force applied to the optical unit connecting pin 42.

Therefore, as shown in Fig. 4 (b), when the vision screener is tilted so that the LCD screen of the display unit of the vision screener can be easily viewed to the examiner, the optical unit 40 also is rotated by the tilting angle of the vision screener and is located to be horizontal with the eye to be examined. Thus, according to the present invention, it is possible to examine the eye in its front direction, and at the same time the LCD screen can be easily observed by the examiner.

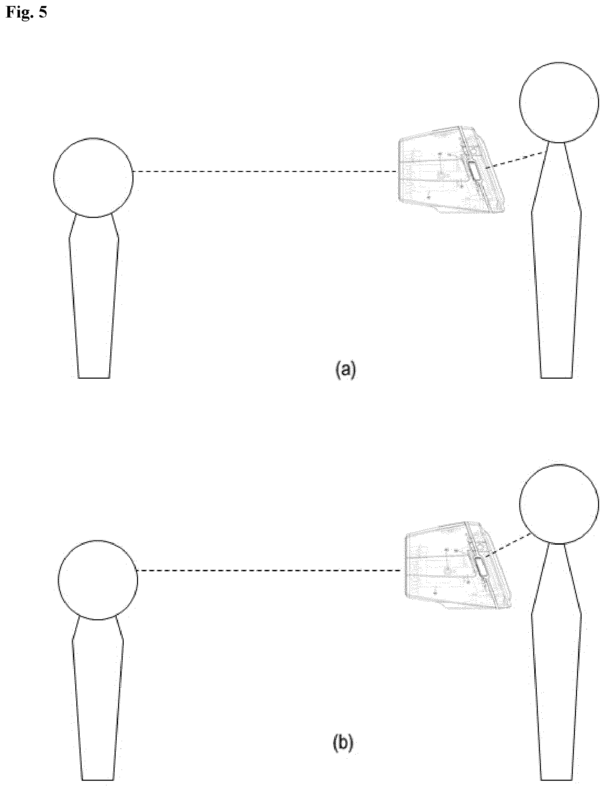

Fig. 5 is a view for illustrating a comparison of the eye levels (directions) of an examiner when the vision screener in accordance with an embodiment of the present invention and the conventional vision screener are used. Specifically, Fig. 5(a) shows the eye level of the examiner when a conventional vision screener is used to match the eye level of the patient. Fig. 5(b) shows the eye level of the examiner when the vision screener of the present invention is used to match the eye level of the patient. The vision screener is generally used for examining a subject of a lower age and a lower height, such as babies and children. The vision screener should be located to be horizontal to the eye of the subject, namely, located at the lower height. In this time, the examiner should bent his/her waist or knees to properly monitor the vision screener located at the lower height.

On the other hand, in the vision screener of the present invention, the vision screener can be tilted while the optical unit 40 is maintained to be horizontal with the eye. The tilting of the optical unit 40 is achieved by a mechanical mechanism using the gyro sensor 10 and the driving motor 20. According to the present invention, the vision screener can be positioned in front of the eye to be examined and at the same time the vision screener can be tilted so that the examiner easily monitors the display part of the vision screener without bending bent his/her waist or knees.

Although the present invention has been described with reference to the specific examples and accompanying drawings, the present invention is not limited to what is shown in the examples and drawings described above. Reference numerals are labeled in the following claims to aid understanding, but the scope of the following claims is not limited to the reference numerals and what is shown in the drawings, and should be construed to encompass all modifications, equivalent constructions and functions of the exemplary embodiments.

## Claims

1. A gyro sensor tilting module of a vision screener, comprising:
a gyro sensor (10) for detecting a tilting angle of the vision screener;
a driving motor (20) for tilting the optical unit (40) by the tilting angle in accordance with the tilting angle detected by the gyro sensor (10); and
the optical unit (40) which is tilted by the driving motor (20) to be aligned horizontally with an eye of a subject to be examined for measuring the eye.

2. The gyro sensor tilting module of claim 1, wherein the driving motor (20) includes a motor shaft (25) which is rotated in accordance with the tilting angle detected by the gyro sensor (10), and a rotation guide (30) mounted to the motor shaft (25) and rotated in accordance with the tilting angle with the motor shaft (25), and
wherein one end of the rotation guide (30) is connected to an optical unit connecting pin (42) which is mounted on the optical unit (40), and the optical unit (40) is rotatably mounted in the vision screener via an optical unit rotation shaft (45).

3. The gyro sensor tilting module of claim 2, wherein, when the rotation guide (30) is rotated, (i) the optical unit connecting pin (42) located at one end of the rotation guide (30) and (ii) the optical unit (40) are also rotated by the tilting angle with a rotation axis of the optical unit rotation shaft (45).

4. The gyro sensor tilting module of claim 3, wherein the optical unit (40) is rotated by the angle of between 0 to 10°.

5. The gyro sensor tilting module of claim 1, wherein, when the vision screener is tilted, the tilting angle of the vision screener is detected with the gyro sensor (10), and a signal corresponding to the detected tilting angle is transmitted to the driving motor (20), and thereby the optical unit (40) is tilted to be aligned horizontally with the eye to be examined.

6. The gyro sensor tilting module of claim 1, further comprising a bearing (47) at each end of the optical unit rotation shaft (45) in order to rotate the optical unit rotation shaft (45) and the optical unit (40) with a force applied to the optical unit connecting pin (42).
